# FASCICULE DE BREVET EUROPEEN

(11) **EP 1 278 486 B1**
(45) Date de publication et mention de la délivrance du brevet: **23.03.2005**
(21) Numéro de dépôt: 00948073.2
(22) Date de dépôt: 03.07.2000
(51) Int. Cl.: A61F 2/44

(54) **CAGE INTERSOMATIQUE D'IMMOBILISATION DU RACHIS**
WIRBELKÄFIG ZUR IMMOBILISIERUNG DER WIRBELSÄULE
INTERBODY SPINAL STABILISATION CAGE

(30) Priorité: 01.07.1999 FR 9908501
(43) Date de publication de la demande: 29.01.2003
(73) Titulaire: Spinevision S.A., 75012 Paris (FR)
(72) Inventeur: BOLGER, Ciaran, Frampton Cotterel, Bristol BS36 2NE (GB); BOLGER, John, Rath Farnham, Dublin16 (IE)
(74) Mandataire: Breese, Pierre
(86) Numéro de dépôt international: PCT/FR2000/001892
(87) Numéro de publication internationale: WO 2001/001894

(56) Documents cités:
- EP-A- 0 951 879
- WO-A-97/06753
- WO-A-97/46165
- WO-A-97/48352
- WO-A-99/42062
- WO-A-99/66867
- DE-C- 4 327 054
- US-A- 5 683 394
- US-A- 5 702 391
- US-A- 5 800 550

## Description

La présente invention concerne le domaine de l'orthopédie du rachis, et plus particulièrement celui des cages intersomatiques.

De telles prothèses sont destinées à immobiliser les segments rachidiens pathologiques et provoquer une consolidation osseuse par la fusion des deux segments. Ces cages sont généralement réalisées en titane, carbone ou matériau composite biocompatible et reçoivent des greffons osseux.

A titre d'exemple, on pourra se reporter à la demande de brevet français FR 2764795 décrivant une cage intersomatique de l'art antérieur. Cette cage comprend un espace creux central destiné à être rempli de fragments d'os. Elle présente, en section transversale, deux faces larges, convexes et pourvues chacune d'une ouverture centrale, deux faces étroites, pourvues chacune d'une ouverture centrale, lesdites faces étroites portant une denture oblique répartie de part et d'autre de l'ouverture centrale des faces étroites. Elle présente par ailleurs une extrémité antérieure fermée et convexe et une extrémité postérieure présentant en son centre un logement polygonal de réception d'un organe de manoeuvre. Une clé de pose pourvue d'un embout polygonal et d'une tige filetée coopère avec la cage de l'invention pour l'introduire par impaction entre les vertèbres et l'immobiliser par rotation après impaction.

Une autre demande de brevet français publié sous le numéro FR2761879 décrit un implant qui comprend, au niveau de ses deux côtés diamétralement opposés destinés à venir au contact de l'os spongieux des vertèbres après implantation, une pluralité d'ouvertures séparées et décalées angulairement les unes par rapport aux autres d'un pas correspondant sensiblement a la moitie de la longueur d'une ouverture, de sorte qu'une de ces ouvertures se trouve en face de la partie de la paroi de l'implant qui sépare deux ouvertures longitudinalement adjacentes.

Une autre demande de brevet FR2717068 décrit une cage intersomatique à insérer par voie antérieure entre deux vertèbres comprenant deux branches sensiblement parallèles pour contact avec les corps vertébraux, un pont reliant des extrémités postérieures des branches et une pièce pour écarter angulairement les deux branches après insertion de la cage entre les deux vertèbres. La cage permet un réglage préopératoire de l'angle de lordose entre les deux vertèbres. Un porte-cage est lié de manière amovible à la cage pour insérer la cage entre les deux vertèbres. Après retrait du porte-cage, un tournevis tourne une vis ayant une extrémité postérieure solidaire de la pièce d'écartement et vissée dans l'une des branches pour écarter les deux branches de cage.

Il a également été proposé dans le brevet américain N° US 5,800,550 de réaliser une cage intersomatique comportant des cylindres à dents qui peuvent être poussés verticalement dans les vertèbres, grâce à un outil muni d'un pas de vis, afin d'assurer un maintien de la cage intersomatique.

Il a également été proposé dans la demande de brevet allemand N° DE 4327054 de réaliser une cage intersomatique comportant un arbre à came permettant de faire monter des pitons de soutien.

Il a également été proposé dans la demande internationale de brevet N° WO 97/06753 de réaliser une cage intersomatique comprenant un corps creux (1, 51) à peu près cylindrique pourvu d'une ou plusieurs ouvertures (9, 10, 59, 59', 60, 60'). Le corps creux (1, 51) peut être élargi de manière ajustable par des éléments d'ajustement (18, 19, 65, 66) et comprend des éléments de fixation (24, 25, 69, 70, 71, 72) qui permettent de le fixer sur des vertèbres.

Il a également été proposé dans la demande internationale de brevet N° WO 97/46165 de réaliser une cage intersomatique comprenant au moins deux vis de base (1), deux pièces de verrouillage (2) empêchant la rotation de la vertèbre, ainsi qu'une pièce de connexion (3, 4, 5).

Le problème qui se pose avec ces cages intersomatiques de l'art antérieur est celui de la fixation provisoire avant que la fusion osseuse ne se soit opérée. Il est en effet vital que la cage reste en place. Les solutions de l'art antérieur ne sont pas satisfaisantes car elles nécessitent un impactage direct ou indirect de la cage dans l'espace intervertébral.

Les inconvénients majeurs des dispositifs de l'art antérieur résident à la fois dans le fait qu'ils ne permettent pas une immobilisation parfaite de la cage intersomatique dans les segments rachidiens, notamment lors des mouvements d'extensions et / ou de rotation de la colonne vertébrale et dans le fait qu'ils ne permettent pas réellement l'ablation de la cage intersomatique.

Le but de la présente invention est de proposer une cage intersomatique améliorée permettant une implantation par la voie cervicale, thoracique ou lombaire, par acte chirurgical antérieur ou postérieur, garantissant une fixation efficace de la cage avant la fusion osseuse et une fois que la fusion osseuse est opérée, et permettant de retirer la cage après fusion osseuse.

Le but de la cage selon l'invention est de permettre de conserver l'espace intervertébral constant afin d'empêcher la distraction des corps vertébraux.

A cet effet, l'invention concerne dans son acception la plus générale une cage intersomatique d'immobilisation du rachis selon la revendication 1.

Un ancrage est, par définition, constitué d'un blocage ne comportant aucun degré de liberté. La cage intersomatique ainsi ancrée par les moyens d'ancrage bloque les vertèbres à la fois en compression, en extension et en rotation, afin de former un tout parfaitement solidaire.

De préférence, le moyen d'entraînement présente une tête d'entraînement en rotation et est mobile en rotation dans les deux sens, afin de permettre la réversibilité des moyens d'ancrage.

Selon le mode de réalisation préféré, lesdits moyens d'ancrage présentent une forme générale en S et le centre de gravité de la forme en S coïncide avec l'axe du moyen d'entraînement.

Avantageusement, chaque moyen d'ancrage comporte au moins une arête coupante radiale sensiblement perpendiculaire à l'axe du moyen d'entraînement, afin de permettre une bonne pénétration des moyens d'ancrage dans la masse osseuse lors de la mise en place de la cage et éventuellement lors de son ablation.

Avantageusement, chaque moyen d'ancrage comporte également au moins une arête coupante en retour sensiblement parallèle à l'arête coupante principale afin de permettre une bonne pénétration des moyens d'ancrage dans la masse osseuse lors de l'ablation de la cage.

Avantageusement également, chaque moyen d'ancrage comporte une lumière, afin de permettre la repousse osseuse entre les bras des moyens d'ancrages. La coopération ainsi obtenue entre les moyens d'ancrage et la repousse osseuse garantie un maintien parfait des vertèbres à la fois en compression, en extension et en rotation.

En outre, les bords de ces lumières sont de préférence au moins partiellement coupants, afin de permettre la réalisation d'une fente pour le dégagement des moyens d'ancrage et l'ablation de la cage intersomatique.

Avantageusement également, la cage selon l'invention comporte des moyens de blocage / déblocage en force des moyens d'ancrage dans leur position ancrée.

Avantageusement enfin, ledit ou lesdits moyens d'entraînement comportent en leurs centres un trou dont les parois sont filetées, afin de permettre de positionner un ou plusieurs éléments supplémentaires, telle qu'une vis de fixation, dans l'axe du ou des moyens d'entraînement.

L'invention sera mieux comprise à la lecture de la description qui suit, se référant à l'exemple non limitatif illustré par les dessins annexés où :

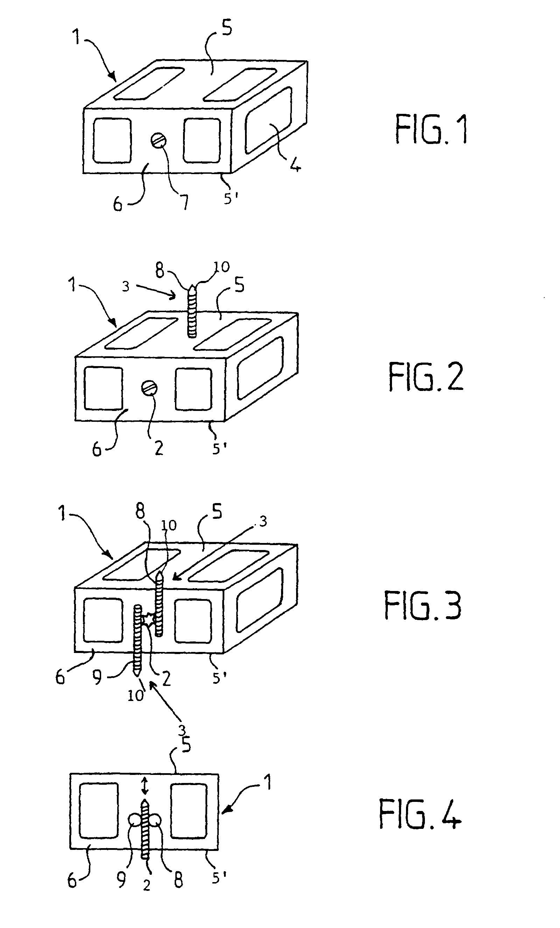

- la figure 1 représente une vue schématique en perspective d'une cage intersomatique ;
- les figures 2, 3 et 4 représentent respectivement une vue schématique en perspective et des vues schématiques en coupe d'une cage intersomatique correspondant à un mode de réalisation des moyens d'ancrage différent de celui de la cage intersomatique selon l'invention ;
- les figures 5 et 6 illustrent respectivement une vue en perspective de deux paires de moyens d'ancrage en S selon le mode de réalisation des moyens d'ancrage de la cage intersomatique selon l'invention ;
- la figure 7 illustre une vue en perspective d'une cage intersomatique selon l'invention à une paire de moyens d'ancrage ;
- les figures 8 et 9 illustrent respectivement une vue de face de la cage intersomatique de la figure 7, en position escamotée et en position d'ancrage ;
- la figure 10 illustre une vue de côté d'une cage intersomatique selon l'invention à une paire de moyens d'ancrage.
- la figure 11 illustre une vue en perspective d'une cage intersomatique selon l'invention à double paire de moyens d'ancrage ; et
- les figures 12 et 13 illustrent respectivement une vue de face de la cage intersomatique de la figure 11, en position d'ancrage et en position ancrée.

La cage intersomatique (1) selon l'invention présente une forme globalement parallélépipédique et délimite un espace intérieur (4) creux s'ouvrant par des lumières prévues sur ces différentes faces et notamment sur ces faces latérales (5, 5') et frontale (6). L'espace intérieur (4) est destiné à être rempli avec des fragments osseux prélevés sur le patient. Ces fragments osseux sont destinés à initier une fusion des vertèbres entre lesquelles la cage intersomatique est placée.

La face frontale (6) présente une lumière permettant d'accéder à la tête (7) d'entraînement en rotation du moyen d'entraînement (2).

Ainsi, grâce au moyen d'entraînement, les moyens d'ancrage présentent chacun deux positions extrêmes : une position escamotée dans laquelle ils sont positionnés à l'intérieur de la cage et une position ancrée dans laquelle ils présentent un débattement maximum en dehors de la cage. Entre ces deux positions extrêmes, les moyens d'ancrage présentent une pluralité de positions d'ancrage intermédiaires.

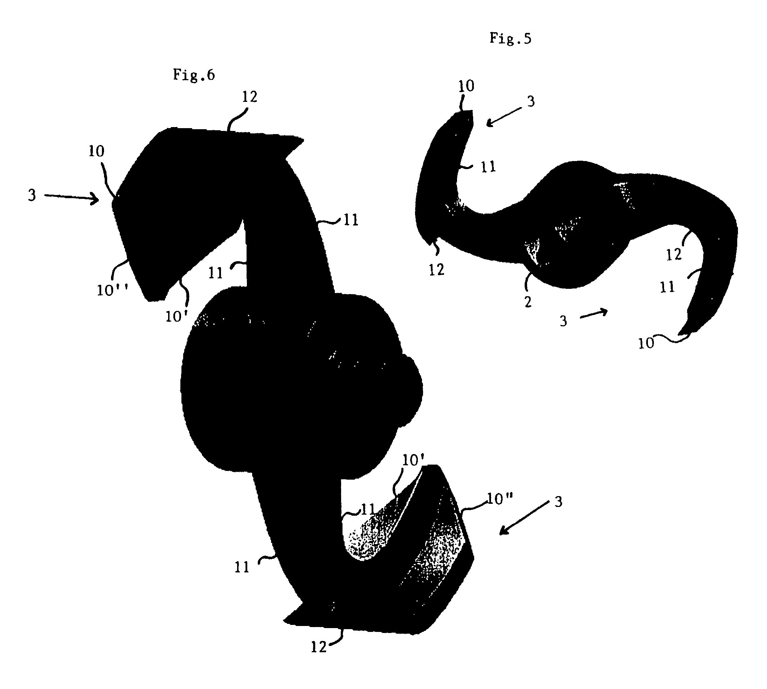

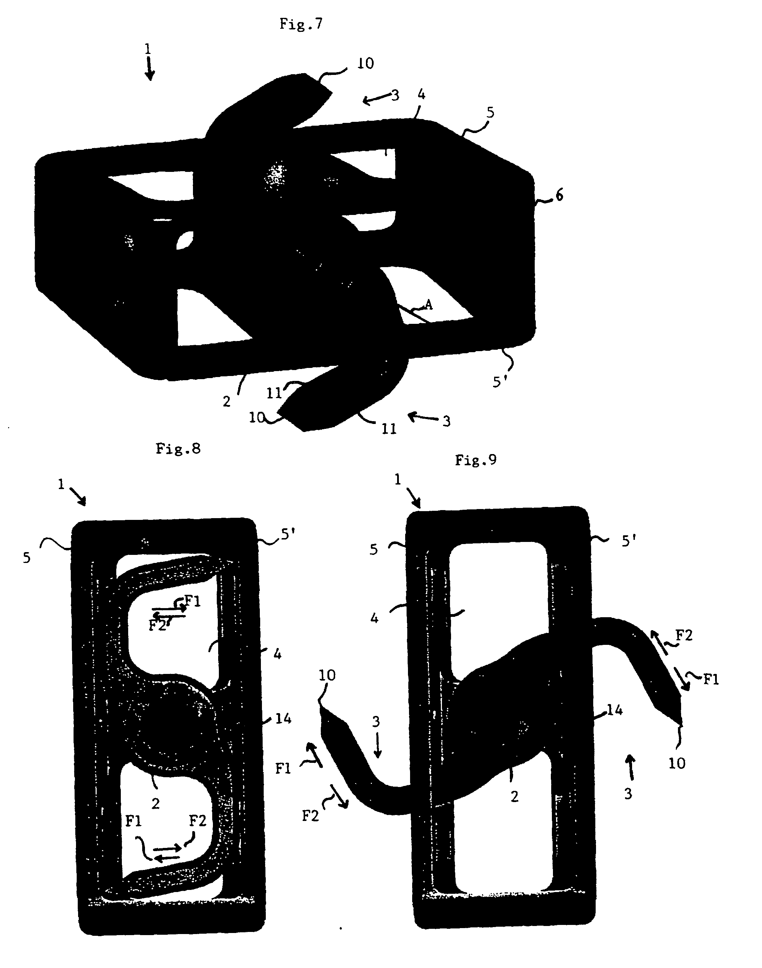

Dans le mode préféré de réalisation de l'invention, lesdits moyens d'ancrage (3) présentent une forme générale en S, comme illustré figures 5 et 6, et le centre de gravité de la forme en S est positionné sur l'axe A du moyen d'entraînement (2), comme illustré figure 7.

Chaque moyen d'ancrage (3) comporte de préférence au moins une arête coupante radiale (11), positionnée sur un rayon du moyen d'ancrage, sensiblement perpendiculaire à l'axe du moyen d'entraînement, sur le bord des bras de liaison des moyens d'ancrage, ainsi qu'au moins une arête coupante en retour (12) sensiblement parallèle à l'arête coupante (10) principale, comme illustré figures 5 et 6.

La figure 6 illustre une version de l'invention dans laquelle ladite arête coupante (10) principale constitue la base d'un triangle au moins isocèle, voire équilatéral, munis de côtés coupants (10', 10'').

Sur cette figure, chaque moyen d'ancrage (3) comporte deux arêtes coupantes radiales (11) positionnées sensiblement sur le même rayon des moyens d'ancrage (3), sur des bords opposés, intérieur et extérieur, des bras de liaison.

Sur la figure 7, chaque moyen d'ancrage (3) comporte deux arêtes coupantes radiales (11) positionnées côte à côte sur la face intérieure des bras de liaison.

Le mouvement de coupe permettant l'ancrage de la cage est illustré par les flèches F1 sur les figures 8 et 9, alors que le mouvement d'ablation est illustré par les flèches F2.

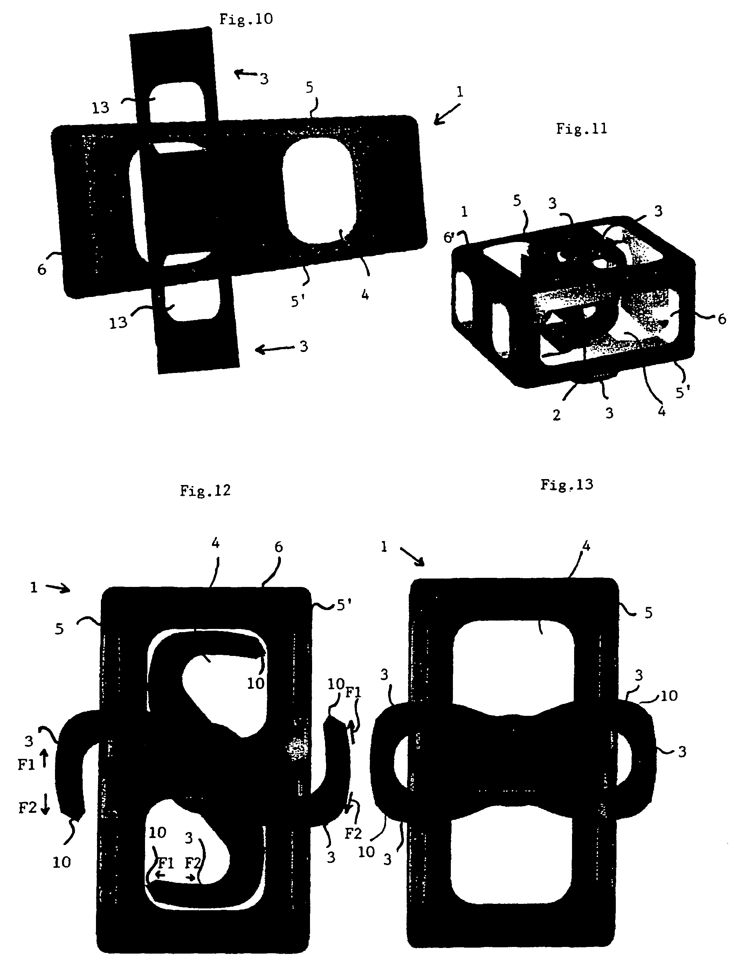

En outre, chaque moyen d'ancrage (3) comporte de préférence une lumière (13) dont les bords sont au moins partiellement coupants, comme illustré figure 10.

Pour permettre de bloquer les moyens d'ancrage (3) en position d'ancrage, la cage selon l'invention comporte de préférence des moyens de blocage /déblocage en force des moyens d'ancrage (3) dans leur position ancrée, constitués, par exemple par un bossage destiné à coopérer avec un évidement.

De préférence, la cage intersomatique (1) selon l'invention, comme illustré figure 11, comporte deux paires de moyens d'ancrage (3) tournant, par exemple dans deux sens opposés, chaque moyen d'entraînement (2) de chaque paire de moyens d'ancrage (3) débouchant dans une même face frontale (6).

Dans cette version, le mouvement de coupe permettant l'ancrage de la cage est illustré par les flèches F1 sur la figure 12, alors que le mouvement d'ablation est illustré par les flèches F2.

La figure 13 illustre la cage intersomatique à double S, en position ancrée.

La cage est avantageusement réalisée en carbone. Un tel matériau présente un module d'Young comparable à celui de l'os. Les moyens d'ancrage sont avantageusement réalisés en hydroxyapatite, ou un matériau lié et fritté de granulés de composé de phosphate de calcium. Les granulés sont liés avec des particules de composé de phosphate de calcium. Les moyens d'ancrage peuvent également être réalisés en alliage métallique à base de titane ou d'inox.

Les moyens d'ancrage peuvent également être réalisés en bicalcium de phosphate, en tricalcium de phosphate ou en tantalum poreux.

L'invention est décrite dans ce qui précède à titre d'exemple. L'homme du métier sera à même de réaliser diverses variantes, notamment pour l'actionnement des moyens d'ancrage et l'érection par l'actionnement d'un organe d'ancrage accessible par une face frontale, compatibles avec les revendications annexées.

## Revendications

1. Cage intersomatique (1) d'immobilisation du rachis présentant une forme parallélépipédique et comprenant un espace creux central (4) destiné à être rempli de fragments d'os, comportant des moyens d'ancrage (3) débouchant sur des faces latérales (5, 5') de liaison avec des vertèbres adjacentes, entraînés par au moins un moyen d'entraînement (2) débouchant dans une face frontale (6), lesdits moyens d'ancrage (3) comportant chacun au moins une arête coupante (10) principale, **caractérisée en ce que** lesdits moyens d'ancrage (3) présentent une forme générale en S.

2. Cage intersomatique (1) selon la revendication principale **caractérisée en ce que** le moyen d'entraînement (2) présente une tête (7) d'entraînement en rotation et est mobile en rotation dans les deux sens, afin de permettre la réversibilité des moyens d'ancrage.

3. Cage intersomatique (1) selon la revendication 1 ou 2, **caractérisée en ce que** le centre de gravité de la forme en S est positionné sur l'axe du moyen d'entraînement (2).

4. Cage intersomatique (1) selon l'une au moins des revendications précédentes **caractérisée en ce que** ladite arête coupante (10) principale constitue la base d'un triangle au moins isocèle munis de côtés coupants (10', 10'').

5. Cage intersomatique (1) selon l'une au moins des revendications précédentes **caractérisée en ce que** chaque moyen d'ancrage (3) comporte au moins une arête coupante radiale (11) positionnée sur un rayon du moyen d'ancrage.

6. Cage intersomatique (1) selon l'une au moins des revendications précédentes **caractérisée en ce que** chaque moyen d'ancrage (3) comporte au moins une arête coupante en retour (12) sensiblement parallèle à l'arête coupante (10) principale.

7. Cage intersomatique (1) selon l'une au moins des revendications précédentes **caractérisée en ce que** chaque moyen d'ancrage (3) comporte une lumière (13).

8. Cage intersomatique (1) selon la revendication 7 **caractérisée en ce que** les bords de ladite lumière (13) sont au moins partiellement coupants.

9. Cage intersomatique (1) selon l'une au moins des revendications précédentes **caractérisée en ce qu'**elle comporte des moyens de blocage / déblocage en force des moyens d'ancrage (3) dans leur position ancrée.

10. Cage intersomatique (1) selon l'une au moins des revendications précédentes, **caractérisée en ce qu'**elle comporte deux paires de moyens d'ancrage (3), chaque moyen d'entraînement (2) de chaque paire de moyens d'ancrage (3) débouchant dans une même face frontale (6).

11. Cage intersomatique (1) selon l'une au moins des revendications précédentes **caractérisée en ce que** ledit ou lesdits moyens d'entraînement (2) comportent en leurs centres un trou (14) dont les parois sont filetées.

## Patentansprüche

1. Intersomatisches Gehäuse (1) zur Blockierung des Rückgrats, wobei das Gehäuse eine parallelepipedische Form aufweist und einen zentralen Hohlraum (4) umfasst, der dazu dient mit Knochenfragmenten gefüllt zu werden, wobei dieses Gehäuse Verankerungsmittel (3) aufweist, die an lateralen Seiten (5, 5') zur Verbindung mit angrenzenden Wirbeln münden, die durch mindestens ein Antriebsmittel (2) angetrieben werden, das in eine frontale Seite (6) mündet, wobei die besagten Verankerungsmittel (3) jeweils mindestens eine scharfe Hauptkante (10) aufweisen, **dadurch gekennzeichnet, dass** die besagten Verankerungsmittel (3) jeweils eine allgemeine S-förmige Form aufweisen.

2. Intersomatisches Gehäuse (1) nach Anspruch 1, **dadurch gekennzeichnet, dass** das Antriebsmittel (2) einen Antriebskopf (7) in Rotation aufweist und in Rotation in beide Richtungen mobil ist, um die Reversibilität der Verankerungsmittel zu erlauben.

3. Intersomatisches Gehäuse (1) nach Anspruch 1 oder 2, **dadurch gekennzeichnet, dass** der Schwerpunkt der S-förmigen Form auf der Achse des Antriebsmittels (2) positioniert ist.

4. Intersomatisches Gehäuse (1) nach mindestens einem der vorhergehenden Ansprüche, **dadurch gekennzeichnet, dass** die besagte scharfe Hauptkante (10) die Basis eines zumindest gleichschenkligen mit scharfkantigen Seiten (10', 10'') versehenen Dreiecks darstellt.

5. Intersomatisches Gehäuse (1) nach mindestens einem der vorhergehenden Ansprüche, **dadurch gekennzeichnet, dass** jedes Verankerungsmittel (3) mindestens eine radiale scharfe Kante (11), die auf einem Radius des Verankerungsmittels positioniert ist, aufweist.

6. Intersomatisches Gehäuse (1) nach mindestens einem der vorhergehenden Ansprüche, **dadurch gekennzeichnet, dass** jedes Verankerungsmittel (3) zumindest eine scharfe Rückkante (12) aufweist, die fast parallel zur scharfen Hauptkante (10) ist.

7. Intersomatisches Gehäuse (1) nach mindestens einem der vorhergehenden Ansprüche, **dadurch gekennzeichnet, dass** jedes Verankerungsmittel (3) eine Leuchte (13) aufweist.

8. Intersomatisches Gehäuse (1) nach Anspruch 7, **dadurch gekennzeichnet, dass** die Ränder von der besagten Leuchte (13) zumindest teilweise schneidend sind.

9. Intersomatisches Gehäuse (1) nach mindestens einem der vorhergehenden Ansprüche, **dadurch gekennzeichnet, dass** es Zwangsblockierungs- /-entblockierungsmittel der Verankerungsmittel (3) in ihrer verankerten Position aufweist.

10. Intersomatisches Gehäuse (1) nach mindestens einem der vorhergehenden Ansprüche, **dadurch gekennzeichnet, dass** es zwei Paare Verankerungsmittel (3) aufweist, wobei jedes Antriebsmittel (2) von jedem Paar Verankerungsmittel (3) in einer selben frontalen Seite (6) mündet.

11. Intersomatisches Gehäuse (1) nach mindestens einem der vorhergehenden Ansprüche, **dadurch gekennzeichnet, dass** das besagte oder die besagten Antriebsmittel (2) in ihren Zentren ein Loch (14) mit Gewindewänden aufweist oder aufweisen.

## Claims

1. Intersomatic cage (1) for immobilisation of rachis with a parallelepiped shape and comprising a central hollow space (4) that will be filled with bone fragments, comprising anchor means (3) opening up on lateral faces (5, 5') connecting to adjacent vertebrae, entrained by at least one driving means (2) opening up in a front face (6), each of the said anchor means (3) comprising at least one main cutting edge (10), **characterised in that** it the said anchor means (3) have a generally S-shape.

2. Intersomatic cage (1) according to the main claim, **characterised in that** the driving means (2) are provided with a drive head (7) in rotation and are free to rotate in both directions, to enable reversibility of the anchor means.

3. Intersomatic cage (1) according to claim 1 or 2, **characterised in that** the centre of gravity of the S shape is positioned on the axis of the driving means (2).

4. Intersomatic cage (1) according to at least one of the above claims, **characterised in that** the said main cutting edge (10) forms the base of a triangle that is at least isosceles with cutting sides (10', 10").

5. Intersomatic cage (1) according to at least one of the above claims, **characterised in that** each anchor means (3) comprises at least one radial cutting edge (11) positioned on a radius of the anchor means.

6. Intersomatic cage (1) according to at least one of the above claims, **characterised in that** each anchor means (3) comprises at least one cutting edge in return (12) approximately parallel to the main cutting edge (10).

7. Intersomatic cage (1) according to at least one of the above claims, **characterised in that** each anchor means (3) is provided with an opening (13).

8. Intersomatic cage (1) according to claim 7, **characterised in that** the edges of the said opening (13) are at least partially cutting.

9. Intersomatic cage (1) according to at least one of the above claims, **characterised in that** it comprises force means of blocking / releasing of anchor means (3) in their anchored position.

10. Intersomatic cage (1) according to at least one of the above claims, **characterised in that** it comprises two pairs of anchor means (3), each driving means (2) of each pair of anchor means (3) opening up in the same front face (6).

11. Intersomatic cage (1) according to at least one of the above claims, **characterised in that** the said entrainment means (2) comprise in their centres a hole (14) with threaded walls.
